# EUROPEAN PATENT SPECIFICATION

(11) **EP 0 816 322 B1**
(45) Date of publication and mention of the grant of the patent: **26.03.2003**
(21) Application number: 97110021.9
(22) Date of filing: 19.06.1997
(51) Int. Cl.: C07C 69/96, C07C 69/734, A61K 7/46, C07D 327/06, C07C 329/06

(54) **Fragrance precursors**
Vorläufer von Duftstoffen
Précurseurs de parfums

(30) Priority: 24.06.1996 EP 96110157; 30.04.1997 EP 97107133
(43) Date of publication of application: 07.01.1998
(73) Proprietor: Givaudan SA, 1214 Vernier-Genève (CH)
(72) Inventor: Anderson, Denise, 8032 Zurich (CH); Frater, Georg, 8400 Winterthur (CH)
(74) Representative: Patentanwälte Schaad, Balass, Menzl & Partner AG

(56) References cited:
- WO-A-95/04809
- US-A- 4 395 370
- US-A- 4 420 472
- A.A.L.CHALLIS AND G.R.CLEMO: "HOMOVANILLIN" JOURNAL OF THE CHEMICAL SOCIETY., 1947, LETCHWORTH GB, pages 1692-1697, XP002042282
- DATABASE XFIRE BEILSTEIN INFORMATIONSSYSTEME GMBH, FRANKFURT BRN=3362012, XP002042287 & SCHVING,SABATAY: BULLETIN DE LA SOCIETE CHIMIQUE DE FRANCE., vol. 43, no. 4, 1928, PARIS FR, page 858
- KAZUYOSHI TAKEDA ET AL.: "A Synthesis of a New Type of Alkoxycarbonylating Reagents from 1,1-Bis(6-(trifluoromethyl)benzotriazolyl) Carbonate (BTBC) and Their Reactions" SYNTHESIS., no. 6, June 1987, STUTTGART DE, pages 557-560, XP002042283
- F. HOULIHAN ET AL.: "Phase transfer catalysis in the tert-butyloxycarbonylation of alcohols,phenols,enols,and thiols with di-tert-butyl dicarbonate" CANADIAN JOURNAL OF CHEMISTRY., vol. 63, no. 1, January 1985, OTTAWA CA, pages 153-162, XP002042284
- JUNJI INANAGA ET AL.: "Organic Synthesis with Trialkylphosphine Catalysts.Conjugate Addition of Alcohols to alpha,beta-Unsaturated Alkinic Acid Esters" CHEMISTRY LETTERS., no. 2, February 1993, TOKYO JP, pages 241-244, XP002042285
- SALVADOR BLAYA ET AL.: "Stereoselective Synthesis of beta-Alkoxy- and beta-Alkylthio-Acrylic Esters and Amides from beta-Tosylacrylic Derivatives" TETRAHEDRON., vol. 51, no. 12, 20 March 1995, OXFORD GB, pages 3617-3626, XP002042286

## Description

The invention relates to fragrance precursors. In particular, the invention relates to the use of several classes of compounds which can act as fragrance precursors e.g. in cosmetic products such as deodorants and antiperspirants and in laundry products such as detergents and fabric softeners. These compounds are normally odorless or nearly so, but upon contacting the skin as for example, in skin care compositions or in personal care compositions, produce fragrances. The compounds also produce fragrances when used in the presence of lipases, e.g. as used in (laundry) detergents, thus providing a prolongation of the fabric scenting effect.

A principal strategy currently employed in imparting odours to consumer products is the admixing of the fragrance directly into the product. There are however, several drawbacks to this strategy. The fragrance material can be too volatile, resulting in fragrance loss during manufacturing, storage, and use. Many fragrance materials are also unstable over time. This again results in loss during storage.

In some cases, fragrances are microencapsulated or treated with cyclodextrins to form inclusion complexes to help decrease volatility and improve stability. However, these methods are for a number of reasons often not successful. In addition, cyclodextrins can be too expensive.

In many consumer products it is desirable for the fragrance to be released slowly over time. Microencapsulation and cyclodextrins have been used to provide slow-release properties, however, they are subject to the same limitations as above.

In WO 95/04809 a method for scenting fabrics being washed in the presence of a lipase-containing detergent, whereafter they are optionally treated with a fabric softener is disclosed. The method is characterized in that a detergent and/or a fabric softener contain a compound which provides a slow release of odoriferous alcohol, aldehyde or ketone.

The present invention now provides compositions comprising at least one compound which show a low level of odour, or are even odourless, prior to application to the skin, but which release odorant molecules after application to the skin (that is, they provide a delayed release of the fragrance), in particular to the skin in the axilla. The compounds of the present invention also release odorant molecules when used in the presence of lipase-containing products, and, this way, provide a prolongation of the fabric scenting effect.

The fragrance precursor composition under consideration are compositions comprising at least one compound of the formula 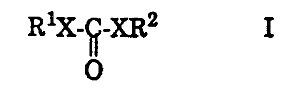 wherein R¹ and R² which can be the same or different, are the radicals of fragrant alcohols or fragrant mercaptans R¹XH and R²XH, and, if only one of the radicals R¹ and R² is a fragrant alcohol, the other radical R¹ or R² is a polyalcohol or a sugar radical, of which one or more of the hydroxyl functions may be substituted as carbonates XR¹ and/or XR² as depicted above, and R³ and R⁴, which are the same or different, are H, C₁₋₆-alkyl or, together, form a carbocyclic or heterocyclic ring, and R⁵ is R¹ or R²,
X=O or S,

Suitably in a cosmetically acceptable carrier.

Examples of alcohols R¹XH or R²XH are primary or secondary alcohols or phenols
- such as:
amyl alcohol
hexyl alcohol*
2-hexyl alcohol*
heptyl alcohol*
octyl alcohol*
nonyl alcohol*
decyl alcohol*
undecyl alcohol*
lauryl alcohol*
myristic alcohol
3-methyl-but-2-en-1-ol*
3-methyl-1-pentanol
cis-3-hexenol*
cis-4-hexenol*
3,5,5-trimethyl hexanol
3,4,5,6,6-pentamethylheptan-2-ol (Kohinool, International Flavors & Fragrances)*
citronellol*
geraniol*
oct-1-en-3-ol
2,5,7-trimethyl octan-3-ol (Corps Abricot, Givaudan-Roure)
2-cis-3,7-dimethyl-2,6-octadien-1-ol
6-ethyl-3-methyl-5-octen-1-ol (Meo Parf, Givaudan-Roure)*
3,7-dimethyl-oct-3,6-dienol*
3,7-dimethyloctanol (Pelargol, Givaudan-Roure)*
7-methoxy-3,7-dimethyl-octan-2-ol (Osyrol, BBA)*
cis-6-nonenol*
5-ethyl-2-nonanol
6,8-dimethyl-2-nonanol (Nonadyl, Givaudan-Roure)*
2,2,8-trimethyl-7 (8)-nonene-3-ol (Corps Lavande, Givaudan-Roure)
nona-2,6-dien-1-ol
4-methyl-3-decen-5-ol (Undecavertol, Givaudan-Roure)*
dec-9-en-1-ol
benzylalcohol
2-methyl undecanol
10-undecen-1-ol
1-phenyl ethanol*
2-phenyl ethanol*
2-methyl-3-phenyl-3-propenol
2-phenyl propanol*
3-phenyl propanol*
4-phenyl-2-butanol
2-methyl-5-phenyl pentanol (Rosaphen, H+R)*
2-methyl-4-phenyl-pentanol (Pamplefleur, International Flavors & Fragrances)*
3-methyl-5-phenyl-pentanol (Phenoxanol, International Flavors & Fragrances)*
2-(2-methylphenyl)-ethanol*
4-(1-methylethyl)benzene methanol
4-(4-hydroxyphenyl)butan-2-one*
2-phenoxy ethanol*
4-(1-methylethyl)-2-hydroxy-1-methyl benzene
2-methoxy-4-methyl phenol
4-methyl phenol
anisic alcohol*
p-tolyl alcohol*
cinnamic alcohol*
vanillin*
ethyl vanillin*
eugenol*
isoeugenol*
thymol
anethol*
decahydro 2-naphthalenol
borneol*
cedrenol (Givaudan-Roure)*
farnesol*
fenchyl alcohol*
menthol*
3,7,11-trimethyl-2,6,10-dodecatrien-1-ol
alpha ionol*
tetrahydro ionol*
2-(1,1-dimethylethyl)cyclohexanol*
3-(1,1-dimethylethyl)cyclohexanol*
4-(1,1-dimethylethyl)cyclohexanol*
4-isopropyl cyclohexanol (Folrosia® Givaudan-Roure)
6,6-dimethyl-bicyclo [3.3.1]hept-2-ene-2-ethanol (Diheptol, Dragoco)
6,6-dimethyl-bicyclo [3.1.1]hept-2-ene-methanol (Myrtenol, BBA)*
p-menth-8-en-3-ol (Isopulegol, Givaudan-Roure)*
3,3,5-trimethyl cyclohexanol
2,4,6-trimethyl-3-cyclohexenyl-methanol*
4-(1-methylethyl)cyclohexyl-methanol (Mayol, Firmenich)*
4-(1,1-dimethylethyl)cyclohexanol
2-(1,1-dimethylethyl)-cyclohexanol (Verdol, International Flavors & Fragrances)
2,2,6-trimethyl-alpha-propyl cyclohexane propanol (Timberol, Dragoco)*
5-(2,2,3-trimethyl-3-cyclopentenyl)-3-methyl pentan-2-ol (Sandalore®Givaudan-Roure)*
3-methyl-5-(2,2,3-trimethyl cyclopentyl-3-enyl)pent-4-en-2-ol(Ebanol, Givaudan-Roure)*
2-ethyl-4(2,2,3-trimethyl cyclopentyl-3-enyl)but-2-en-1-ol (Radjanol, Givaudan-Roure)*
4-(5,5,6-trimethylbicyclo[2.2.1] hept-2-yl)-cyclohexanol (Sandela, Givaudan-Roure)*
2-(2-methylpropyl)-4-hydroxy-4-methyl-tetrahydropyran* (Florosa Q, Quest)*
2-cyclohexyl propanol*
2-(1,1-dimethylethyl)-4-methyl cyclohexanol (Rootanol, BASF)*
1-(2-tert-butyl-cyclohexyloxy)-2-butanol (Amber Core, Kao)*
1-(4-isoporpyl-cyclohexyl)-ethanol (Mugetanol, H&R)*
etc.

* preferred alcohols

Examples of thiols R¹XH or R²XH are:
3-mercapto-1-hexanol
2-(1-mercapto-1-methylethyl)-5-methylcyclohexanone methoxy-4-methyl-2-butane-2-thiol
thiogeraniol
thioterpineol.

It is a matter of course, that it is not possible to give a complete list of the odoriferous alcohols and mercaptans R¹XH and R²XH, which alcohols and mercaptans are liberated as a result of the desired cleavage of the carbonates I by bacteria, in particular axilla bacteria, or lipases and which alcohols are then capable of imparting agreeable odours.

The skilled artisan is, however, quite aware of those alcohols and mercaptans, which provide a positive contribution to the fragrance compositions.

Examples of polyalcohols are diols, e.g.
diethylene glycol, propylene glycol, triethylene glycol, polyglycols, triols, e.g. glycerol, etc.

Examples of sugars are furanoside and pyranoside sugars, e.g glucose, fructose, etc.

The compounds I may preferably be used as sustained release odorants but also to mask or attenuate undesirable odours or to provide additional odours not initially present in consumer products, i.e. cosmetic products destined for application to human skin such as underarm deodorants or antiperspirants or other deodorants contacting the body, or in hand lotions, baby powders, baby lotions, ointments, foot products, facial cleansers, body wipes, facial make-up, colognes, after-shave lotions, shaving creams, etc. Additional applications include laundry detergents, fabric softeners, fabric softener sheets, automatic dishwasher detergents, and other lipase-containing consumer products.

The compounds I are virtually odourless under normal temperature and atmospheric conditions, i.e. about 10 - 50 degrees Celsius and about 20 to 100 % relative humidity. However, when applied to the body or when used in an application in the presence of lipases, they undergo a transformation in which the fragrant alcohol is released.

The compounds I are not limited to any particular isomers, all possible stereo- as well as geometric isomers as well as mixtures thereof are thus included within the scope of formula I.

The compounds I, upon cleavage, provide alcohols having organoleptic properties and therefore permit the development of methods useful in enhancing the odour of consumer products. These compounds may be used individually in an amount effective to enhance the characteristic odour of a material. More commonly, however, the compounds are mixed with other fragrance components in an amount sufficient to provide the desired odour characteristics.

The amount required to produce the desired, overall effect varies depending upon the particular compounds I chosen, the product in which it will be used, and the particular effect desired.

For example, depending upon the selection and concentration of the compound chosen, when I is added either singly or as a mixture e.g. to a deodorant or laundry product composition at levels ranging from about 0.1 to about 10 % by weight, or most preferred about 0.25 to about 4 % by weight, an odorant, i.e. an odoriferous alcohol in an "organoleptically effective amount" is released when the product is used. This newly formed odorant serves to enhance the odour of the fragrance.

The compounds I can accordingly be used in the manufacture of odorant compositions used in the preparation of cosmetic and laundry products e.g. deodorants, antiperspirants, laundry detergents, fabric softeners, and as is evident from the above compilation, a broad range of known odorants or odorant mixtures can be used. In the manufacture of such compositions the known odorants or odorant mixtures set forth above can be used according to methods known to the perfumer, such as e.g. from W.A. Poucher, Perfumes, Cosmetics, Soaps, 2, 7th Edition, Chapman and Hall, London 1974.

The compounds I can be prepared by using standard methods known to the skilled chemist. These standard methods can be found in the chemical literature. For example, carbonates can be synthesized by reaction of a carbonic acid equivalent, phosgene, or a chemical equivalent of phosgene, with one or more alcohols or mercaptans in the presence of base. Alternatively, reaction of a chloroformate which is another such carbonic acid equivalent and an alcohol or mercaptan R¹XH or R²XH, in the presence of base, also yields a carbonate. This reaction is the substitution of a chloroformate by R¹X or R²X; see Comprehensive Organic Chemistry, Vol. 2 D. Barton, W. D. Ollis, Ed. p. 1070.

The vinylogous carbonates can be prepared by β-addition of mercaptans or alcohols to propiolic acid esters, preferentially catalysed by tertiary amines, such as trimethylamine or triethylamine, etc.

Convenient methods are outlined in the Examples.

### Example 1

### Carbonic acid bis - (2-phenyl-ethyl)ester

To a solution of 10.72 g triphosgene in 80 ml dichloromethane, 26.51 g phenethyl alcohol was added. While cooling in an ice bath, 17.16 g pyridine was dropped in over 45 minutes. Then the reaction was stirred at room temperature for 62 hours. The reaction was diluted with dichloromethane, washed with aqueous HCl and aqueous NaHCO₃. The organic phase was dried, filtered and evaporated to dryness. The resulting crystals were recrystallized from 60 ml hexanes and then a second time from hexane: methyl t.-butyl ether (50 ml : 50 ml) to yield 15.48 g of colourless crystals.
- NMR (CDCl₃) δ: 7,34 - 7,18 (m,10H), 4,31 (t, J=7,18 Hz,4H), 3,00 (t, J= 7,17 Hz,4H).

### Example 2

### Carbonic acid 2,3-bis-hex -3-enyloxycarbonyloxy-propyl ester hex-3-enyl ester

To a solution of 32.30 g triphosgene in 100 ml dichloromethane 34.45 g cis-3-hexenol was dropped in over 10 minutes while cooling in an ice/salt-bath. Then 26.46 g pyridine was dropped in over 1 h 45 min. After stirring the reaction for 21 hours at room temperature it was diluted with 200 ml pentane, filtered and evaporated to dryness to yield 51.14 g of a yellow oil. The raw product was then dropped in an ice cooled solution of 4.8 g glycerine and 30 ml pyridine in 100 ml dichloromethane over 1 h 45 min. After stirring the reaction for 48 h at room temperature, it was diluted with 200 ml ether and washed with 2 x 200 ml HCl 2N. The water phase was extracted with ether, then the combined organic phases were washed with NaHCO₃ and brine, dried and evaporated to dryness. The residue was purified by distilling off starting material first and then silica gel chromatography to yield 19.5 g of an oil.
- NMR (CDCl₃) δ: 5,60 - 5,25 (m,6H), 5,16 - 5,06 (m,1H), 4,47 - 4,23 (m,4H), 4,18 - 4,07 (m,6H), 2,48 - 2,37 (m,6H), 2,13 - 1,99 (m,6H), 0,97 (t, J=7,48 Hz, 9H).

According to the same procedure, the following compound was prepared:

### Example 3

### Carbonic acid 2,3-bis-phenethyloxycarbonyloxy-propyl ester phenethyl ester

Starting from phenethyl alcohol and glycerol;
- NMR (CDCl₃) δ: 7,35 - 7,20 (m,15H), 5,13 - 5,03 (m,1H),4,41 4,19 (m,10H), 2,97 (t, J=7,17 Hz,6H).

### Example 4

### Carbonic acid benzyl ester phenethyl ester

To a mixture of 29.96 g phenethyl alcohol and 30 ml pyridine in 150 ml dichloromethane, 60.5 g benzylchloroformate was dropped in over 1 h 45 min while cooling in an ice-bath. After stirring over night at room temperature, the reaction was diluted with ether, washed with 2N HCl, NaHCO₃ and water. After extraction with ether, the combined organic layers were dried and evaporated to dryness. The residue was purified by silica gel chromatography to yield the product: 52.5 g of a colourless oil.
- NMR (CDCl₃) δ: 7,37 - 7,16 (m,10H), 5,13 (s,2H), 4,34 (t, J=7,17 Hz, 2H), 2,96 (t, J=7,17 Hz, 2H).

According to the same procedure, the following compounds were prepared:

### Example 5

### Carbonic acid benzyl ester hex-3-enyl ester

Starting from cis-3-hexenol and benzylchloroformate;
- NMR (CDCl₃) δ: 7,41 - 7,29 (m,5H), 5,58 - 5,44
(m,2H), 5,15 (s, 2H),
4,13 (t, J=7,02, 2H),
2,47 - 2,36 (m,2H), 2,11-1,97
(m,2H), 0,95 (t, J=7,5 Hz,3H).

### Example 6

### Carbonic acid benzyl ester dec-9-enyl ester

Starting from dec-9-en-1-ol and benzyl chloroformate;

### Example 7

### Carbonic acid 4-allyl-2-methoxy-phenyl ester benzyl ester

Starting from Eugenol and benzyl chloroformate;
- NMR (CDCl₃) δ: 7,44 - 7,26 (m,5H), 7,04 - 6,70 (m,3H), 6,03 - 5,83 (m,1H), 5,24 (s,2H), 5,14 - 5,02 (m,2H), 3,75 (s,3H), 3,34 (d, J=6,71 Hz,2H).

### Example 8

### Carbonic acid hex-3-enyl ester 2-(2-hex-3-enyloxy-carbonyloxy-ethoxy)-ethyl ester

Starting from cis-3-hexenol and diethylene glycol-bis-chloroformate;
- NMR (CDCl₃) δ: 5,59 - 5,25 (m,4H), 4,30 - 4,25 (m,4H), 4,12 (t, J=7,01 Hz,4H), 3,78 - 3,70 (m,4H), 2,47 - 2,37 (m,4H), 2,13 - 1,99 (m,4H), 0,97 t, J=7,63 Hz,6H).

### Example 9

### Carbonic acid 3,7-dimethyl-oct -6-enyl ester 2-[2-(3,7-dimethyl-oct-6-enyloxycarbonyloxy)-ethoxy]-ethyl ester

Starting from citronellol and diethylene glycol-bis-chloroformate;
- NMR (CDCl₃) δ: 5.12-5.04 (m, 2H), 4.30-4.14 (m, 8H), 3.75-3.70 (m, 4H), 2.04-1.91 (m, 4H), 1.74-1.15 (m, 22H), 0.92 (d, J=6.5 Hz, 6H).

### Example 10

The following sulfur compounds were prepared:
Thiocarbonic acid 0-ethyl ester S-[1-methyl-1-(4-methyl-2-oxo-cyclohexyl)-ethyl] ester from thio-dihydro-carvon and chloro formic acid ethylester.
4-Propyl-[1,3]oxathian-2-one from 3-mercapto-hexanol-1 and phosgene.
Carbonic acid 3-ethoxycarbonylsulfanyl-hexyl ester ethyl ester from 3-mercapto-hexanol-1 and chloro formic acid ethyl ester.

Additionally, the compounds listed below were prepared:

### Example 11

| **R**^{**1**}**OH** | **Synthesis via Example x from:** | **Product** |
|---|---|---|
| cis-3-hexenol | x = 1 cis-3-hexenol; triphosgene | Carbonic acid dihex-3-enyl ester |
| citronellol | x = 1 citronellol; triphosgene | Carbonic acid bis-(3,7-dimethyl-oct-6-enyl)ester |
| Rosalva (dec-9-en-1-ol) | x = 1 dec-9-en-1-ol; triphosgene | Carbonic acid didec-9-enyl ester |
| phenylethyl alcohol | x = 4 phenylethyl alcohol; 4-tert-butyl-cyclohexyl chloroformate | Carbonic acid 4-tert-butyl-cyclohexyl ester phenethyl ester |
| geraniol | x = 4 geraniol; 4-tert-butyl-cyclohexyl chloroformate | Carbonic acid 4-tert-butyl-cyclohexyl ester 3,7-dimethyl-octa-2,6-dienyl ester |
| geraniol | x = 8 geraniol; diethylene glycol-bis-chloroformate | Carbonic acid 3,7-dimethyl-octa-2,6-dienyl ester 2-[(3,7-dimethyl-octa-2,6-dienyloxy-carbonyloxy)ethoxyl-ethyl ester |
| phenylethyl alcohol | x = 4 phenylethyl alcohol; butyl chloroformate | Carbonic acid butyl ester phenethyl ester |
| cis-3-hexenol | x = 4 cis-3-hexenol; butyl chloroformate | Carbonic acid butyl ester hex-3-enyl ester |
| geraniol | x = 4 geraniol; butyl chloroformate | Carbonic acid butyl ester 3,7-dimethyl-oct-2,6-dienyl ester |
| benzyl alcohol | x = 4 1,6-hexanediol; benzyl chloroformate | Carbonic acid benzyl ester 6-benzyloxycarbonyloxy-hexyl ester |
| ethyl vanillin | x = 7 ethyl vanillin; benzyl chloroformate | Carbonic acid benzyl ester 2-ethoxy-4-formylphenyl ester |

### Example 12

Test cloth was washed with a lipase-containing detergent to which one or more delayed release fragrances had been added. Headspace analysis of the wet and dry laundry indicated the presence of the fragrant alcohols. The alcohol level was higher than when the test cloth was washed with a lipase-containing detergent to which one or more fragrant alcohols were added.

### Example 13

Test cloth was washed with a lipase-containing detergent and then a fabric softener, containing one or more delayed release fragrances, was added to the rinse cycle. Headspace analysis of the wet and dry laundry indicated the presence of the fragrant alcohols. The alcohol level was higher than when the test cloth was washed with a lipase-containing detergent and then a fabric softener, containing one or more delayed fragrant alcohols, was added to the rinse cycle.

### Example 14

Axilla bacteria cultures containing 0.1 % precursor I were incubated for 20 hours at 30 °C. After filtration from the cells, the presence of the parent alcohol was in each case detected by headspace-GC techniques and/or the majority of an 18 member panel.

The same tests were carried out with inactivated cultures (85°/20 min). The odour of the parent alcohols could not be detected after incubation, excluding therefore a hydrolysis by the medium or the culture.

### Example 15

The following set forth examples for the use of the delayed release fragrances of the present invention in various products. The methods of forming the following compositions are well known to those skilled in the art. All formulations may contain additional ingredients known to those skilled in the art, e.g. colorants, opacifiers, buffers, antioxidants, vitamins, emulsifiers, UV absorbers, silicones and the like. All products can also be buffered to the desired pH. All values are % w/w.

| **Deo-colognes** | | | | |
|---|---|---|---|---|
| Delayed Release Fragrances | 0.5 | 1.5 | 2.5 | 6.0 |
| Fragrance | 0.5 | 1.5 | 2.5 | 6.0 |
| Triclosan Ciba Geigy) | 1.0 | - | 0.75 | 1.0 |
| Alcohol to | 100 | 100 | 100 | 100 |

### Deo-Sticks:

| **Antiperspirant** | |
|---|---|
| Ethylene Glycol Monostearate | 7.0 |
| Shea butter | 3.0 |
| Neobee 1053 (PVO International) | 12.0 |
| Generol 122 (Henkel) | 5.0 |
| Kesscowax B (Akzo) | 17.0 |
| Dimethicone Dow Corning 345 | 35.0 |
| Aluminum Sesquichlorhydrate | 20.0 |
| Delayed Release Fragrances | 0.5 |
| Fragrance | 0.5 |

| **Antiperspirant** | |
|---|---|
| Steary Alcohol | 17.0 |
| Castor Wax | 3.0 |
| Talc | 5.0 |
| Aluminum Zirconium | |
| Tetrachlorhydrate | 20.0 |
| Delayed Release Fragrances | 1.0 |
| Fragrance | 1.0 |
| Dimethicone Dow 245 | to 100.0 |

| **Clear Deodorant Stick** | |
|---|---|
| Witconol APM | 43.0 |
| Propylene Glycol | 20.0 |
| Alcohol 39C | 20.0 |
| Demin water | 7.0 |
| Monamid 150ADD | 5.0 |
| Millithix 925 | 2.0 |
| Ottasept Extra | 0.5 |
| Delayed Release Fragrances | 0.75 |
| Fragrance | 0.75 |

| **Deodorant Stick** | |
|---|---|
| Propylene Glycol | 69.0 |
| Demin Water | 21.8 |
| Triclosan | 0.2 |
| Sodium Stearate | 8.0 |
| Delayed Release Fragrances | 0.5 |
| Fragrance | 0.5 |

| **Alcohol free Deodorant Stick** | |
|---|---|
| PPG-3 Myristyl Ether (Witconol APM) | 36.0 |
| Propylene Glycol | 36.0 |
| Demin Water | 19.0 |
| Triclosan | 0.25 |
| Sodium Stearate | 7.75 |
| Delayed Release Fragrances | 0.5 |
| Fragrance | 0.5 |

| **Antiperspirant Aerosol** | |
|---|---|
| Absolute Ethanol | 15.0 |
| Zirconium Aluminum tetrachlorhydrate | 5.0 |
| Bentone 38 | 1.5 |
| Delayed Release Fragrances | 0.75 |
| Fragrance | 0.75 |
| S-31 Hydocarbon propellant to | 100.0 |

| **Antiperspirant Pump** | |
|---|---|
| Demin water | 57.5 |
| Aluminum Sesquichlorhydrate | 20.0 |
| Triton X-102 (Union Carbide) | 2.0 |
| Dimethyl Isosorbide (ICI) | 20.0 |
| Delayed Release Fragrances | 0.25 |
| Fragrance | 0.25 |

| **Roll-On** | |
|---|---|
| Dimethicone DC 354 (Dow Corning) | 69.0 |
| Bentone 38 | 10.0 |
| Rezal 36 GP ( Reheis Chem. Co.) | 20.0 |
| Delayed Release Fragrances | 0.5 |
| Fragrance | 0.5 |

In the above, the following components were used:

| | |
|---|---|
| Triclosan | 5-chloro-2-(2,4-dichlorophenoxy)phenol |
| Neobee 1053 | glycerol tricaprate/caprylate |
| Generol 122 | soya sterol |
| Kesscowax B | cetyl alcohol and glycol polymer |
| Witconol APM | polypropylene glycol-3 myristyl ether |
| Monamid 150 ADD | cocoamide diethanolamine |
| Millithix 925 | dibenzylidene sorbitol |
| Ottasept Extra | quaternium 18 hectorite |
| Bentone 38 | quaternium 18 hectorite |
| Triton X-102 | octoxynol-13 |
| Dimethicone DC 354 | mixture of fully methylated linear siloxanepolymers end-blocked with trimethylsiloxy units |
| Rezal 36 GP | Aluminium zirconium tetrachlorohydrexglycine |

## Claims

1. A fragrance precursor composition comprising at least one compound of the formula 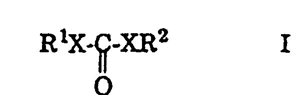 wherein R¹ and R² which can be the same or different, are the radicals of fragrant alcohols or fragrant mercaptans R¹XH and R²XH, and, if only one of the radicals R¹ and R² is a fragrant alcohol, the other radical is a polyalcohol or a sugar radical, of which one or more of the hydroxyl functions may be substituted as carbonates XR¹ and/or XR² as depicted above,
X = O or S
suitably in a cosmetically acceptable carrier.

2. The composition according to claim 1, wherein the compound of formula I is selected from the group consisting of
Carbonic acid bis-(2-phenyl-ethyl)ester,
Carbonic acid 2,3-bis-hex-3-enyloxycarbonyloxy-propyl ester hex-3-enyl ester,
Carbonic acid 2,3-bis-phenethyloxycarbonyloxy-propyl ester phenethyl ester,
Carbonic acid benzyl ester phenethyl ester,
Carbonic acid benzyl ester hex-3-enyl ester,
Carbonic acid benzyl ester dec-9-enyl ester,
Carbonic acid 4-allyl-2-methoxy-phenyl ester benzyl ester,
Carbonic acid hex-3-enyl ester 2-(2-hex-3-enyloxy-carbonyloxy-ethoxy)-ethyl ester,
Carbonic acid 3,7-dimethyl-oct-6-enyl ester 2-[2-(3,7-dimethyl-oct-6-enyloxycarbonyloxy)-ethoxyl-ethyl ester,
Thiocarbonic acid 0-ethyl ester S-[1-methyl-1-(4-methyl-2-oxo-cyclohexyl)-ethyl]ester,
4-Propyl-[1,3]oxathian-2-one,
Carbonic acid 3-ethoxycarbonylsulfanyl-hexyl ester ethyl ester,
Carbonic acid dihex-3-enyl ester,
Carbonic acid bis-(3,7-dimethyl-oct-6-enyl)ester,
Carbonic acid didec-9-enyl ester,
Carbonic acid 4-tert-butyl-cyclohexyl ester phenethyl ester,
Carbonic acid 4-tert-butyl-cyclohexyl ester 3,7-dimethyl-octa-2,6-dienyl ester,
Carbonic acid 3,7-dimethyl-octa-2,6-dienyl ester 2-[(3,7-dimethyl-octa-2,6-dienyloxycarbonyloxy)ethoxyl-ethyl ester,
Carbonic acid benzyl ester 6-benzyloxycarbonyloxy-hexyl ester and
Carbonic acid benzyl ester 2-ethoxy-4-formylphenyl ester.

3. A fragrance precursor product containing at least one compound I as defined in claim 1, or 2.

4. A process for prolonging the effect of diffusion of the caracteristic odour of an odoriferous compound R¹XH and/or R²XH comprising applying a composition as defined in claim 1 or 2.

5. A method of suppressing human body malodour by means of compounds of the formula I as defined in claim 1, which comprises the application to human skin of a fragrance precursor product as defined in claim 3.

6. Use of the fragrance precursor composition as defined in claim 1 or 2 in consumer products, laundry detergents, fabric softeners, fabric softener sheets, automatic dishwasher detergents, and other lipase-containing consumer products.

## Revendications

1. Composition de précurseur de parfum comprenant au moins un composé de formule : 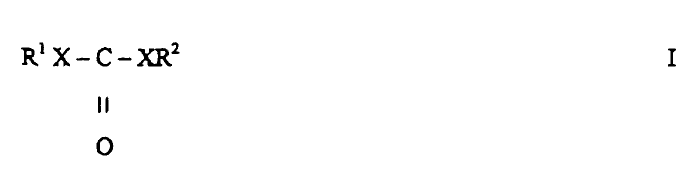 dans laquelle R¹ et R² qui peuvent être identiques ou différents, sont des radicaux d'alcools parfumés ou de mercaptans parfumés R¹ XH et R² XH, et, seulement si l'un des radicaux R¹ et R² est un alcool parfumé, l'autre radical est un polyalcool ou un radical de sucre dont une ou plusieurs des fonctions hydroxy peut être substituée par des carbonates XR¹ et/ou XR² comme décrit ci-dessus,
X=O ou S
de façon appropriée dans un matériau formant support acceptable.

2. Composition selon la revendication 1, dans laquelle le composé de formule I est sélectionné à partir du groupe constitué de :
bis - (2 - phényl - éthyl) ester d'acide carbonique,
2,3 - bis - hex - 3 - ényloxycarbonyloxy - propyl ester hex - 3 ényl - ester d'acide carbonique,
2,3 - bis - phénétyloxycarbonyloxy - propyl ester phénétyl ester d'acide carbonique,
benzyl ester phénétyl ester d'acide carbonique,
benzyl ester hex - 3 - ényl ester d'acide carbonique,
benzyl ester dec - 9 - ényl ester d'acide carbonique,
4 - allyl - 2 - méthoxy - phényl ester benzyl ester d'acide carbonique,
hex- 3- ényl ester 2 - (2 - hex - 3 - ényloxy - carbonyloxy - éthoxy) - éthyl ester d'acide carbonique,
3,7 - diméthyl - oct - 6 - ényl ester 2 - [2 - (3,7 - diméthyl - oct - 6 - ényloxycarboyloxy) - éthoxy] - éthyl ester d'acide carbonique,
0 - éthyl ester S - [1 - méthyl - 1 - (4 - méthyl - 2 - oxo - cyclohéxyl) - éthyl] ester d'acide thiocarbonique,
4-propyl-[1,3]oxathian- 2 - one,
3 - éthoxycarbonylsulfanyl - héxyl ester éthyl ester d'acide carbonique, dihex - 3 - ényl ester d'acide carbonique,
bis - (3,7 - diméthyl - oct - 6 - ényl) ester d'acide carbonique, didec - 9 - ényl ester d'acide carbonique,
4 - tert - butyl - cyclohéxyl ester phénétyl ester d'acide carbonique,
4 - tert - butyl - cyclohéxyl ester 3,7 - diméthyl - octa - 2,6 - diényl ester d'acide carbonique,
3,7 - diméthyl octa - 2,6 - diényl ester 2 - [(3,7 - diméthyl - octa - 2,6 - diényloxycarbonyloxy) éthoxy] - éthyl ester d'acide carbonique,
benzyl ester 6 - benzyloxycarbonyloxy - hexyl ester d'acide carbonique et
benzyl ester 2 - éthoxy - 4 - formylphényl ester d'acide carbonique.

3. Produit précurseur de parfum comprenant au moins un composé de formule I tel que défini dans la revendication 1 ou 2.

4. Procédé pour prolonger les effets de diffusion des caractéristiques d'odeur d'un composé odoriférant R¹ XH et/ou R² XH comprenant le fait d'appliquer une composition selon la revendication 1 ou 2.

5. Procédé pour éliminer les mauvaises odeurs provenant du corps humain par des composés de formule I comme défini dans la revendication 1, qui comprend l'application sur la peau humaine d'un produit précurseur de parfum comme défini dans la revendication 3.

6. Utilisation de la composition de précurseur de parfum tel que défini dans la revendication 1 ou 2 dans des produits destinés aux consommateurs, les détergents pour la lessive, les adoucissants pour les tissus, les feuilles adoucissantes pour les tissus, les détergents pour les lave-vaisselle automatiques, et autres produits de consommation contenant de la lipase.

## Patentansprüche

1. Duftstoffvorläuferzusammensetzung enthaltend mindestes eine Verbindung der Formel 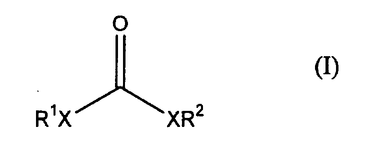 in der R¹ und R², die gleich oder verschieden sein können, Reste eines Duftstoffalkohols oder eines Duftstoffmercaptans R¹XH und R²XH, und, wenn nur einer der Reste R¹ und R² ein Duftstoffalkohol ist, ist der andere Rest ein Polyalkohol oder ein Zuckerrest, von welchem eine oder mehrere der Hydroxylgruppen als Carbonate XR¹ und/oder XR² wie oben beschrieben substituiert sein können,
X= O oder S,
geeignet in einem kosmetisch annehmbaren Träger.

2. Die Zusammensetzung nach Anspruch 1, in der die Verbindung der Formel I ausgewählt ist aus der Gruppe von
Carbonsäure bis-(2-phenyl-ethyl)ester,
Carbonsäure 2,3-bis-hex-3-enyloxycarbonyloxy-propyl ester hex-3-enyl ester,
Carbonsäure 2,3-bis-phenethyloxycarbonyloxy-propyl ester phenethyl ester,
Carbonsäurebenzylester phenethylester,
Carbonsäurebenzylester hex-3-enyl ester,
Carbonsäurebenzylester dec-9-enyl ester,
Carbonsäure 4-allyl-2-methoxy-phenylester benzylester,
Carbonsäure hex-3-enylester 2-(2-hex-3-enyloxycarbonyloxy-ethoxy)-ethyl ester,
Carbonsäure 3,7-dimethyl-oct-6-enyl ester 2-[2-(3,7-dimethyl-oct-6-enyloxycarbonyloxy)-ethoxylethylester,
Thiocarbonsäure O-ethyl ester S-[1-methyl-1-(4-methyl-2-oxo-cyclohexyl)-ethyl]ester, 4-Propyl-[1,3]oxathian-2-on,
Carbonsäure 3-ethoxycarbonylsulfanyl-hexylester ethyl ester,
Carbonsäure dihex-3-enyl ester,
Carbonsäure bis-(3,7-dimethyl-oct-6-enyl)ester,
Carbonsäure didec-9-enyl ester,
Carbonsäure 4-tert-butyl-cyclohexyl ester phenethyl ester,
Carbonsäure 4-tert-butyl-cyclohexyl ester 3,7-dimethyl-octa-2,6-dienylester,
Carbonsäure 3,7-dimethyl-octa-2,6-dienylester 2-[(3,7-dimethyl-octa-2,6-dienyloxycarbonyloxy)ethoxy]ethyl ester,
Carbonsäurebenzylester 6-benzyloxycarbonyloxy-hexyl ester und
Carbonsäurebenzylester 2-ethoxy-4-formylphenylester.

3. Duftstoffvorläuferprodukt enthaltend mindestens eine Verbindung I wie in Anspruch 1 oder 2 definiert.

4. Verfahren zur Verlängerung des Diffusionseffektes des charakteristischen Duftes einer duftenden Verbindung R¹XH und/oder R²XH beinhaltend Auftragen einer Zusammensetzung wie in Anspruch 1 oder 2 definiert.

5. Verfahren zur Unterdrückung von menschlichem Schweiss durch Verbindungen der Formel I wie in Anspruch 1 definiert, das die Anwendung eines Duftstoffproduktes wie in Anspruch 3 definiert, auf menschliche Haut beinhaltet.

6. Verwendung der Duftstoffvorläuferzusammensetzung wie in Anspruch 1 oder 2 definiert, in Konsumgütern, Waschdetergentien, Textilweichspülern, Textilweichspülerblätter, Geschirrspülmaschinendetergentien und anderen Lipasen-haltigen Konsumgütern.
